# FASCICULE DE BREVET EUROPEEN

(11) **EP 2 315 583 B1**
(45) Date de publication et mention de la délivrance du brevet: **09.05.2018**
(21) Numéro de dépôt: 09784269.4
(22) Date de dépôt: 16.07.2009
(51) Int. Cl.: A61K 9/48, A61K 31/05

(54) **FORMULATION DESTINÉE À AMÉLIORER LA BIODISPONIBILITÉ D'UNE MOLÉCULE HYDROPHOBE**
FORMULIERUNG ZUR ERHÖHUNG DER BIOVERFÜGBARKEIT EINES HYDROPHOBEN MOLEKÜLS
FORMULATION FOR IMPROVING THE BIOAVAILABILITY OF A HYDROPHOBIC MOLECULE

(30) Priorité: 18.07.2008 FR 0804098
(43) Date de publication de la demande: 04.05.2011
(73) Titulaire: Yvery, 13008 Marseille (FR)
(72) Inventeur: PECHERE, Laurent, F-13008 Marseille (FR)
(74) Mandataire: Santarelli
(86) Numéro de dépôt international: PCT/FR2009/000868
(87) Numéro de publication internationale: WO 2010/007252

(56) Documents cités:
- EP-A- 1 234 571
- EP-A- 1 236 472
- EP-A- 1 844 784
- WO-A-01/30336
- WO-A-2004/071490
- WO-A1-98/30205
- WO-A2-01/08671
- WO-A2-02/24152
- CA-A1- 2 170 748

## Description

La présente invention se place dans le domaine des formulations destinées à améliorer l'absorption intestinale d'une molécule hydrophobe autrement appelée lipophile.

Par absorption intestinale d'une molécule hydrophobe, on entend selon le présent texte la capacité de ladite molécule lipophile à passer la barrière gastro-intestinale sans être au préalable métabolisée et à se retrouver disponible dans la circulation.

Une molécule est dite hydrophobe ou lipophile quand elle est soluble dans les corps gras, mais insoluble dans l'eau. Une molécule hydrophobe n'a pas la capacité de créer des liaisons hydrogène avec les molécules d'eau. Elle est aussi souvent dite apolaire, ou de faible polarité, ce qui signifie qu'elle ne peut pas créer d'interactions électrostatiques avec l'eau. En effet, la solubilité d'une molécule dans un solvant dépend de manière générale des interactions qu'elle peut avoir avec le solvant. Une molécule hydrophobe est donc une molécule qui ne peut pas interagir physiquement avec l'eau. Elle est alors généralement plutôt soluble dans les solvants organiques.

Actuellement les principes actifs (PA) en développement sont le plus souvent apolaires et appartiennent aux classes II (peu solubles mais perméables) et IV (peu solubles et faiblement perméables) de la classification biopharmaceutique [Amidon et al., G.L., 1995, Pharm. Res. 12:413-420.].

Ces caractéristiques sont défavorables à l'obtention d'une absorption satisfaisante par voie orale.

Ainsi le besoin persiste d'une formulation galénique augmentant l'absorption des molécules hydrophobes. C'est un des buts de la présente invention.

Parmi les molécules hydrophobes, les polyphénols sont des composés que l'on retrouve à l'état naturel dans les végétaux de la classe des spermatophytes et particulièrement dans la vigne. On trouve de tels composés comme par exemple le resvératrol dans le raisin et dans le vin.

Parmi les polyphénols, on trouve les hydroxystilbènes. Dans l'art antérieur les hydroxystilbènes sont utilisés entre autres comme agents dépigmentants (JP87-192040), comme agents vasodilatateurs (EP 96-830517), comme agents antithrombiques (JP 05016413), dans le traitement de diverses affections cardiovasculaires (CA 2187990), comme agents inhibiteurs de mutagenèse et de carcinogenèse (JP 06024967), ou encore décrits comme antioxydants.

Le resvératrol (3,5,4'-trihydroxystilbène) est une phytoalexine polyphénolique. Ce composé est synthétisé par les plantes et agit comme antifongique en réponse à des infections (Bothrytis cinerea). Le resvératrol se trouve dans diverses plantes comme les conifères, les cacahuètes, la peau de raisin rouge, certaines légumineuses et le Polygonum cuspidatum.
Le resvératrol possède des vertus thérapeutiques connues depuis longtemps par les médecines traditionnelles chinoises et japonaises. Le resvératrol a été impliqué comme responsable de la diminution des risques cardiovasculaires appelé le "French Paradox". En effet, il a été établi une corrélation entre la consommation de vin rouge contenant de forts taux de resvératrol, et la diminution de maladies coronariennes. De nombreuses études scientifiques ont mis en évidence que le resvératrol est un antagoniste du récepteur des arylhydrocarbures et de la dioxine (AhR) (Casper, R. F., et coll., Mol. Pharmacol. 1999, 56, 784-790).

Le resvératrol possède aussi des activités antioxydantes, anti-inflammatoires, ostéoprotectrices et pourrait avoir un effet préventif dans certains cancers.

Des modèles in vivo ont été utilisés pour étudier l'absorption du resvératrol. Chez le rat, des études cinétiques ont été réalisées à différents temps après absorption de vin rouge. Les résultats indiquent qu'un pic d'absorption est détecté 60 minutes après ingestion. Après une courte période, le resvératrol est détecté dans le foie et les reins (maximum micromolaire une heure après absorption). L'élimination est très rapide avec les reins comme organe privilégié d'élimination. D'autres études mettent en évidence que le resvératrol sérique apparaît au bout de 15 minutes et diminue très rapidement au bout de 30 minutes. Des travaux similaires ont été réalisés chez la souris. Le resvératrol semble absorbé au maximum par le duodénum et l'élimination du resvératrol est très rapide avec un maximum d'absorption à 30 minutes.

Il ressort de ces études que le resvératrol est très rapidement absorbé, métabolisé au cours du cycle entérohépatique et éliminé.

De nombreuses études ex vivo et in vitro ont porté sur l'absorption et le métabolisme du resvératrol pour connaître la localisation du resvératrol dans les divers organes après son administration et les métabolites produits.

Les modèles ex vivo sont représentés par des perfusions de petit intestin de rat. Ce type d'étude indique que le resvératrol est extrait du petit intestin dans un taux de 46%, 21% se retrouvant au niveau vasculaire et seulement 2% étant retrouvé au niveau intestinal. Ce resvératrol est libre à 40% alors que 11% est glucurono conjugué et 3% est sous forme sulfatée. La forme glucuronide est celle se trouvant dans le système circulatoire alors que la forme sulfatée est la forme sécrétée dans la partie luminale intestinale. Ce même type d'étude a été réalisé dans des modèles d'iléon et de colon perfusé.

Les résultats indiquent que seule une infime partie de resvératrol se retrouve non métabolisé. Des études sur des modèles similaires de foie ou d'intestin humains indiquent que le resvératrol est également très vite métabolisé. Le resvératrol est donc absorbé et métabolisé au niveau intestinal. Lorsque le resvératrol passe dans la circulation, il est pris largement en charge par des protéines sériques.

Les études in vitro utilisant des lignées cellulaires (lignée intestinale CaCo-2) ont confirmé les résultats obtenus sur les modèles intestinaux perfusés.

Enfin, depuis 2003, des études de métabolisme et de biodisponibilité ont été réalisées chez l'homme. Les principales données indiquent que le resvératrol atteint une concentration sérique maximale au bout de 30 minutes. Au delà de 30 minutes, il est rare de trouver du resvératrol inchangé non métabolisé. Dans les meilleurs cas, 2% du resvératrol se trouve au niveau du plasma sous forme inchangée!

Toutes ces données suggèrent que l'activité du resvératrol comme agent thérapeutique sera étroitement liée à sa biodisponibilité qui résulte de son absorption intestinale et de la rapidité de son métabolisme.

Une des manières d'augmenter la concentration de resvératrol sérique inchangée est de mettre au point une forme galénique lui permettant d'augmenter son absorption et donc sa biodisponibilité. C'est un des buts de la présente invention. On pourrait utiliser une formulation destinée à améliorer l'absorption, avantageusement l'absorption intestinale, des polyphénols, comprenant au moins un polyéthylèneglycol ou un de ses équivalents fonctionnels et au moins un éther glycolique ou un de ses équivalents fonctionnels. L'invention revendiquée a pour objet une formulation destinée à la voie orale comprenant un hydroxystilbène répondant à la formule 1 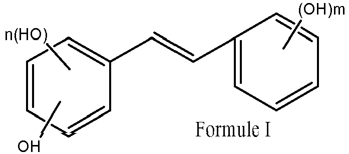 dans laquelle n est un nombre entier compris entre 0 et 4 inclusivement et m est un nombre entier compris entre 0 et 5 inclusivement, sous une forme Cis ou Trans, ainsi que leurs dérivés hydroxyalkylés, au moins un polysorbate et au moins du polyglycéryl-3-dioléate pour améliorer l'absorption intestinale dudit hydroxystilbène.

Une formulation comprenant du resvératrol, un polyéthylèneglycol et un ether glycolique a été divulguée dans le document WO01/30336, mais pour traiter les problèmes cutanés, par une utilisation topique. Le document WO2004/071490 divulgue quant à lui une formulation comprenant du polyéthylène glycol, un éther glycolique et du KOH, dans le but d'augmenter la biodisponibilité de principes actifs pharmaceutiques acides peu solubles. Or il est décrit dans la littérature qu'une telle formulation ne peut pas être utilisée pour augmenter la disponibilité des polyphénols, puisqu'elle détruit les polyphénols (Nardini M., Cirillo E., Natella F., Mencarelli D., Comisso A, Scaccini S., Food Chemistry, vol. 79, issue 1, oct. 2002, p 119-124).

Par "polyphénols" on entend les polyphénols naturels et de synthèse.

Par "polyphénol de synthèse" on entend plus spécifiquement tout polyphénol obtenu par synthèse chimique et non pas par extraction de matériau biologique (plantes) ainsi que tout dérivé d'un polyphénol naturel modifié par substitution ou addition d'atomes à la structure naturelle. Avantageusement, ces substitutions sont des halogènes (Cl-, CF3-) ou des radicaux de structure générale R-O- où R est une chaine aliphatique ou un cycle aromatique ou un radical nitré.

Par "équivalents fonctionnels" on entend un composé qui, mélangé avec un composé polyphénol, exerce sur celui-ci les mêmes effets qu'un polyéthylèneglycol et/ou un éther glycolique. La formulation de l'invention comprend, comme équivalent fonctionnel du polyéthylèneglycol, un polysorbate.

De même, la formulation de l'invention comprend, comme équivalent fonctionnel de l'éther glycolique, le polyglycéryl-3-dioléate. On entend par polyéthylène glycol tout polymère répondant à la formule H(OCH₂CH₂)ₙOH où n est supérieur à trois. A cet égard on citera à titre d'exemple les polyéthylèneglycols de poids moléculaire moyen compris entre environ 100 et 20000, préférentiellement de poids moléculaire moyen compris entre environ 400 et environ 10000, très préférentiellement de poids moléculaire moyen compris entre environ 400 et environ 600.

Un polyéthylèneglycol d'un poids moléculaire donné peut être utilisé seul ou encore en mélange en toute proportion avec un ou plusieurs autres polyéthylène glycols de poids moléculaire variés ou autres équivalents fonctionnels.

Les polyéthylèneglycols peuvent se présenter à la température ambiante soit sous forme liquide, soit sous forme semi-solide selon leur poids moléculaire. En conséquence, ces polymères peuvent être sélectionnés de manière appropriée selon que la formulation destinée à améliorer l'absorption des polyphénols doit se présenter sous forme liquide ou au contraire semi-solide.

L'éther glycolique peut être choisi parmi des éthers de diéthylèneglycol tels que par exemple les alkyléthers de diéthylèneglycol, particulièrement les (C1-C4) alkyléthers de diéthylèneglycol, choisi parmi le méthyléther de diéthylèneglycol, l'éthyléther de diéthylèneglycol, les propylyléthers de diéthylèneglycol ou les butyléthers de diéthylèneglycol, très particulièrement les mono-(C1- C4) alkyléthers de diéthylèneglycol choisi parmi le monométhyléther de diéthylèneglycol, le monoéthyléther de diéthylèneglycol, les monopropylyléthers de diéthylèneglycol ou les monobutyléthers de diéthylèneglycol.

Parmi les alkyléthers de diéthylèneglycol, on préfère les éthers méthylique et éthylique, notamment le diéthylèneglycol monoéthyl éther.

Les éthers glycoliques peuvent être utilisés seuls ou associés en toutes proportions à un (ou plusieurs) autre(s) éther(s) glycolique(s) et/ou à un (ou plusieurs) autre(s) équivalent(s) fonctionnel(s). Selon un mode préféré, la formulation de l'invention destinée à améliorer l'absorption intestinale des hydroxystilbènes comprend un polysorbate en une proportion comprise entre 20 et 97 %, préférentiellement entre 40 et 97 % en poids du poids total de la formulation.

Selon encore un mode préféré de l'invention, la formulation destinée à améliorer l'absorption intestinale des hydroxystilbènes comprend du polyglycéryl-3-dioléate en une proportion comprise entre 2 et 79 %, préférentiellement entre 2 et 59% en poids du poids total de la formulation.

Une formulation selon l'invention particulièrement préférée comprendra entre 50 et 93% de polysorbate, entre 3 et 46% de polyglycéryl-3-dioléate et une quantité suffisante d'eau pour atteindre 100%.

Selon une variante de l'invention, la formulation peut comprendre en outre au moins un émulsifiant. Avantageusement selon l'invention l'émulsifiant peut être un Polysorbate, encore plus avantageusement un polysorbate choisi parmi le Polysorbate 20 (Tween 20 ou de polyoxyéthylène (20) monolaurate de sorbitane), le Polysorbate 40 (Tween 40 ou de polyoxyéthylène (20) Monopalmitate de sorbitane), le Polysorbate 60 (Tween 60 ou de polyoxyéthylène (20) monostéarate de sorbitan), ou le Polysorbate 80 (Tween 80 ou de polyoxyéthylène (20) Monooléate de sorbitane).

La formulation selon l'invention est adaptée pour être utilisée pour améliorer l'absorption intestinale des hydroxystilbènes comme par exemple ceux de formule (I), 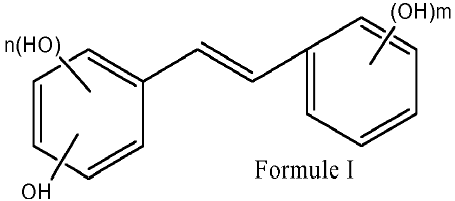 dans laquelle n est un nombre entier compris entre 0 et 4 inclusivement et m est un nombre entier compris entre 0 et 5 inclusivement. Ces composés peuvent être sous une forme Cis ou Trans. Selon l'invention, le terme hydroxystilbène recouvre aussi bien les composés de formule I que leurs dérivés hydroxyalkylés.

Parmi les hydroxystilbènes, on peut citer les mono, di, tri, tétra, penta, hexa, hepta, octo, nonahydroxystilbènes, ou encore leurs dérivés hydroxyalkylés, dont par exemple le 4'-hydroxystilbène, le 2',4'-dihydroxystilbène, le 3',4'-dihydroxystilbène, le 4,4'-dihydroxystilbène, le 2',4',4-trihydroxystilbène, le 3',4',4-trihydroxystilbène, le 2,4,4'-trihydroxystilbène, le 3,4,4'-trihydroxystilbène, le 3,5,4',-trihydroxystilbène, le 2',3,4-trihydroxystilbène, le 2,3',4-trihydroxystilbène, le 2',2,4'-trihydroxystilbène, le 2,4,4',5-tétrahydroxystilbène, le 2',3,4',5-tétrahydroxystilbène, le 2,2',4,4'-tétrahydroxystilbène, le 3,3',4',5-tétrahydroxystilbène, le 2,3',4,4'-tétrahydroxystilbène, le 3,3',4,4'-tétrahydroxystilbène, le 3,3',4',5, 5'-pentahydroxystilbène, le 2,2',4,4',6-pentahydroxystilbène, le 2,3',4,4',6-pentahydroxystilbène, le 2,2',4,4',6,6'-hexahydroxystilbène.

Préférentiellement, on utilise selon l'invention du 3,5,4'-trihydroxystilbène ou resvératrol.

Dans une composition préférée selon l'invention, destinée à la voie orale, on privilégie l'utilisation d'un support ingérable. La composition selon l'invention peut prendre la forme de dragées, gélules, gels, émulsion, comprimés, capsules ou autres formes galéniques utilisables per os. Ces formes sont réalisées par les procédés usuels connus de l'homme du métier.

Selon un mode de réalisation particulier de l'invention, la composition peut être formulée sous forme encapsulée de manière à améliorer significativement la durée de vie du principe actif. Les exemples qui suivent sont donnés à titre illustratif et non limitatif. Dans ce qui suit ou ce qui précède, les proportions sont données en pourcentage pondéral, sauf indications contraires.

### Exemple 1 (hors invention) :

### On prépare la composition suivante qui se présente sous forme liquide :

| | |
|---|---|
| Transcutol P® | 25 mg |
| PEG 600 | 455 mg |
| Resvératrol | 20 mg |

Le resvératrol est dissout en présence du Transcutol P et du PEG 600 qui ont été pesés préalablement. L'ensemble est mis sous agitation jusqu'à obtention d'une phase liquide translucide et claire. Puis on l'encapsule dans des capsules de gélatine conformes à l'utilisation pharmaceutique ou alimentaire.

### Exemple 2 (hors invention) : Mesure du taux sanguin de resvératrol après ingestion.

Une capsule telle que préparée à l'exemple 1 est ingérée par un volontaire dont on a préalablement prélevé un échantillon de sang. Un second échantillon de sang du volontaire est prélevé 4 heures après ingestion.

Les échantillons de sang sont alors analysés par chromatographie en phase liquide couplée à un spectrographe de masse selon les protocoles habituels.

On détermine ainsi le taux sanguin du resvératrol avant et après ingestion.

L'essai est réalisé sur 3 volontaires différents.

| Individus | Concentration sanguine en resvératrol en µg/L | | Taux d'augmentation de la concentration |
|---|---|---|---|
| | T = 0 h | T = 4 h | |
| 1 | 79 | 285 | 3,6 |
| 2 | 23 | 396 | 17,2 |
| 3 | 29 | 295 | 10,4 |

Ces essais démontrent que le resvératrol est encore présent en une concentration nettement élevée 4 heures après ingestion de la composition préparée à l'exemple 1.

### Exemple 3 (hors invention) : Mesure du taux sanguin de resvératrol après ingestion.

Deux capsules telles que préparées à l'exemple 1 sont ingérées par 5 volontaires dont on a préalablement prélevé un échantillon de sang. Un second échantillon de sang des volontaires est prélevé 30 minutes après ingestion. Un troisième échantillon de sang est prélevé 5 heures après ingestion. Un autre individu ingère 40mg de resvératrol sous forme classique (gélule).
Les échantillons de sang sont alors analysés par chromatographie en phase liquide couplée à un spectrographe de masse selon les protocoles habituels.
On détermine ainsi le taux sanguin du resvératrol avant et après ingestion.
Le tableau ci-dessous indique les valeurs moyennes de concentration.

| | Concentration sanguine moyenne en resvératrol (nM) | | |
|---|---|---|---|
| | T = 0 h | T = 30 min | T = 5 h |
| Formule classique gélule | 0 | 422 | 0 |
| Formule capsule (PEG + Transcutol) | 0 | 5295 | 491 |

Ces essais démontrent que le resvératrol est encore présent en une concentration nettement élevée 5 heures après ingestion de la formule contenant du PEG et du Transcutol.

## Revendications

1. Formulation destinée à la voie orale comprenant un hydroxystilbène répondant à la formule 1 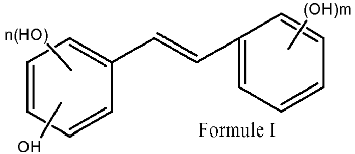 dans laquelle n est un nombre entier compris entre 0 et 4 inclusivement et m est un nombre entier compris entre 0 et 5 inclusivement, sous une forme Cis ou Trans, ainsi que leurs dérivés hydroxyalkylés, au moins un polysorbate et au moins du polyglycéryl-3-dioléate pour améliorer l'absorption intestinale dudit hydroxystilbène.

2. Formulation selon la revendication 1, **caractérisée en ce que** le polysorbate est en une proportion comprise entre 20 et 97 %, en poids du poids total de ladite formulation.

3. Formulation selon l'une quelconque des revendications 1 ou 2, **caractérisée en ce que** le polyglycéryl-3-dioléate est en une proportion comprise entre 2 et 79 %, en poids du poids total de ladite formulation.

4. Formulation selon l'une quelconque des revendications 1 à 3, **caractérisée en ce que** la formulation comprend entre 50 et 93 % en poids du poids total de ladite formulation de polysorbate, 3 et 46 % en poids du poids total de ladite formulation de polyglycéryl-3-dioléate et une quantité suffisante d'eau pour atteindre 100 %.

5. Formulation selon l'une quelconque des revendications 1 à 4, **caractérisée en ce que** dans la formulation le polysorbate est choisi parmi le Polyoxyéthylène (20) monolaurate de sorbitane, le Polyoxyéthylène (20) monopalmitate de sorbitane, le Polyoxyéthylène (20) monostéarate de sorbitane, ou le Polyoxyéthylène (20) monooléate de sorbitane.

6. Formulation selon l'une quelconque des revendications 1 à 5, **caractérisée en ce que** l'hydroxystilbène est un mono, di, tri, tétra, penta, hexa, hepta, octo, nonahydroxystilbène, ou encore leurs dérivés hydroxyalkylés, préférentiellement le 4'-hydroxystilbène, le 2',4'-dihydroxystilbène, le 3',4'-dihydroxystilbène, le 4,4'-dihydroxystilbène, le 2',4',4-trihydroxystilbène, le 3',4',4- trihydroxystilbène, le 2,4,4'-trihydroxystilbène, le 3,4,4'-trihydroxystilbène, le 3,4',5-trihydroxystilbène, le 2',3,4-trihydroxystilbène, le 2,3',4-trihydroxystilbène, le 2',2,4'-trihydroxystilbène, le 2,4,4',5-tétrahydroxystilbène, le 2',3,4',5- tétrahydroxystilbène, le 2,2',4,4'-tétrahydroxystilbène, le 3,3',4',5- tétrahydroxystilbène, le 2,3',4,4'-tétrahydroxystilbène, le 3,3',4,4'-tétrahydroxystilbène, le 3,3', 4',5, 5'-pentahydroxystilbène, le 2,2',4,4',6-pentahydroxystilbène, le 2, 3',4,4',6-pentahydroxystilbène, le 2,2',4,4',6,6'-hexahydroxystilbène, très préférentiellement le 3,4',5-trihydroxystilbène ou resvératrol.

## Patentansprüche

1. Formulierung, bestimmt für den oralen Weg, die ein Hydroxystilben gemäß Formel 1 umfasst: 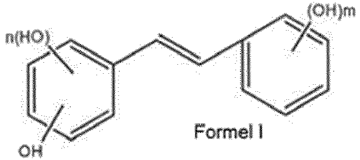 wobei n eine ganze Zahl zwischen 0 und 4 einschließlich und m eine ganze Zahl zwischen 0 und 5 einschließlich ist, in cis- oder trans-Form, sowie deren hydroxyalkylierte Derivate, wenigstens ein Polysorbat und wenigstens Polyglyceryl-3-dioleat, zum Verbessern der intestinalen Resorption des genannten Hydroxystilbens.

2. Formulierung nach Anspruch 1, **dadurch gekennzeichnet, dass** das Polysorbat in einem Anteil zwischen 20 und 97 Gew.-% des Gesamtgewichts der genannten Formulierung vorliegt.

3. Formulierung nach Anspruch 1 oder 2, **dadurch gekennzeichnet, dass** das Polyglyceryl-3-dioleat in einem Anteil zwischen 2 und 79 Gew.-% des Gesamtgewichts der genannten Formulierung vorliegt.

4. Formulierung nach einem der Ansprüche 1 bis 3, **dadurch gekennzeichnet, dass** die Formulierung 50 bis 93 Gew.-% des Gesamtgewichts der genannten Polysorbatformulierung, 3 bis 46 Gew.-% des Gesamtgewichts der genannten Formulierung von Polyglyceryl-3-dioleat und eine ausreichende Menge Wasser enthält, um 100 % zu erreichen.

5. Formulierung nach einem der Ansprüche 1 bis 4, **dadurch gekennzeichnet, dass** die Polysorbatformulierung ausgewählt ist aus Polyoxyethylen (20) Sorbitanmonolaurat, Polyoxyethylen (20) Sorbitanmonopalmitat, Polyoxyethylen (20) Sorbitanmonostearat oder Polyoxyethylen (20) Sorbitanmonooleat.

6. Formulierung nach einem der Ansprüche 1 bis 5, **dadurch gekennzeichnet, dass** das Hydroxystilben ein Mono-, Di-, Tri-, Tetra-, Penta-, Hexa-, Hepta-, Octo-, Nonahydroxystilben oder deren hydroxyalkylierte Derivate ist, vorzugsweise 4'-Hydroxystilben, 2',4'-Dihydroxystilben, 3',4'-Dihydroxystilben, 4,4'-Dihydroxystilben, 2',4',4-Trihydroxystilben, 3',4',4-Trihydroxystilben, 2,4,4'-Trihydroxystilben, 3,4,4'-Trihydroxystilben, 3,4',5-Trihydroxystilben, 2',3,4-Trihydroxystilben, 2,3',4-Trihydroxystilben, 2',2,4'-Trihydroxystilben, 2,4,4',5-Tetrahydroxystilben, 2',3,4',5-Tetrahydroxystilben, 2,2',4,4'-Tetrahydroxystilben, 3,3',4',5-Tetrahydroxystilben, 2,3',4,4'-Tetrahydroxystilben, 3,3',4,4'-Tetrahydroxystilben, 3,3',4',5, 5'-Pentahydroxystilben, 2,2',4,4',6-Pentahydroxystilben, 2,3',4,4',6-Pentahydroxystilben, 2,2',4,4',6,6'-Hexahydroxystilben, äußerst bevorzugterweise 3,4',5-Trihydroxystilben oder Resveratrol.

## Claims

1. Formulation intended for the oral route comprising a hydroxystilbene corresponding to formula 1 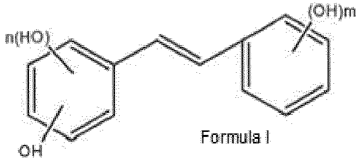 in which n is an integer comprised between 0 and 4 inclusive, and m is an integer comprised between 0 and 5 inclusive, in cis or trans form, as well as their hydroxyalkyl-containing derivatives, at least one polysorbate and at least polyglyceryl-3-dioleate for improving the intestinal absorption of said hydroxystilbene.

2. Formulation according to claim 1, **characterized in that** the polysorbate is in a proportion comprised between 20 and 97% by weight of the total weight of said formulation.

3. Formulation according to any one of claims 1 or 2, **characterized in that** the polyglyceryl-3-dioleate is in a proportion comprised between 2 and 79% by weight of the total weight of said formulation.

4. Formulation according to any one of claims 1 to 3, **characterized in that** the formulation comprises between 50 and 93% by weight of the total weight of said polysorbate formulation, 3 and 46% by weight of the total weight of said polyglyceryl-3-dioleate formulation and a sufficient quality of water to reach 100%.

5. Formulation according to any one of claims 1 to 4, **characterized in that** in the formulation the polysorbate is chosen from polyoxyethylene (20) sorbitan monolaurate, polyoxyethylene (20) sorbitan monopalmitate, polyoxyethylene (20) sorbitan monostearate, or polyoxyethylene (20) sorbitan monooleate.

6. Formulation according to any one of claims 1 to 5, **characterized in that** the hydroxystilbene is a mono, di, tri, tetra, penta, hexa, hepta, octo, nonahydroxystilbene, or also their hydroxyalkyl-containing derivatives, preferentially 4'-hydroxystilbene, 2',4'-dihydroxystilbene, 3',4'-dihydroxystilbene, 4,4'-dihydroxystilbene, 2',4',4-trihydroxystilbene, 3',4',4-trihydroxystilbene, 2,4,4'-trihydroxystilbene, 3,4,4'-trihydroxystilbene, 3,4',5-trihydroxystilbene, 2',3,4-trihydroxystilbene, 2,3',4-trihydroxystilbene, 2',2,4'-trihydroxystilbene, 2,4,4',5-tetrahydroxystilbene, 2',3,4',5-tetrahydroxystilbene, 2,2',4,4'-tetrahydroxystilbene, 3,3',4',5-tetrahydroxystilbene, 2,3',4,4'-tetrahydroxystilbene, 3,3',4,4'-tetrahydroxystilbene, 3,3',4',5, 5'-pentahydroxystilbene, 2,2',4,4',6-pentahydroxystilbene, 2,3',4,4',6-pentahydroxystilbene, 2,2',4,4',6,6'-hexahydroxystilbene, very preferentially 3,4',5-trihydroxystilbene or resveratrol.
